# FASCICULE DE BREVET EUROPEEN

(11) **EP 3 270 968 B1**
(45) Date de publication et mention de la délivrance du brevet: **06.03.2024**
(21) Numéro de dépôt: 16714789.1
(22) Date de dépôt: 18.03.2016
(51) Int. Cl.: A61K 45/06, A61K 31/24, A61P 31/16, A61K 31/554, A61K 31/215, A61K 31/137, A61K 31/704, A61K 31/7056, A61P 31/04, A61P 31/12, A61P 43/00, A61K 31/35

(54) **NOUVELLES COMPOSITIONS ANTIVIRALES POUR LE TRAITEMENT DE LA GRIPPE**
NEUARTIGE ANTIVIRALE ZUSAMMENSETZUNGEN ZUR BEHANDLUNG DER GRIPPE
NOVEL ANTIVIRAL COMPOSITIONS FOR TREATING THE FLU

(30) Priorité: 19.03.2015 FR 1552284
(43) Date de publication de la demande: 24.01.2018
(73) Titulaire: Université Claude Bernard Lyon 1, 69625 Villeurbanne Cedex (FR); Institut National de la Santé et de la Recherche Médicale (INSERM), 75013 Paris (FR); Hospices Civils De Lyon, 69002 Lyon (FR); Université Laval, Québec G1V 0A6 (CA)
(72) Inventeur: ROSA-CALATRAVA, Manuel, 69003 Lyon (FR); TERRIER, Olivier, 69008 Lyon (FR); TEXTORIS, Julien, 69100 Villeurbanne (FR); BOIVIN, Guy, Quebec G1V 1E7 (CA); PIZZORNO, Mario, Quebec G1S 3K6 (CA)
(74) Mandataire: Regimbeau
(86) Numéro de dépôt international: PCT/EP2016/056036
(87) Numéro de publication internationale: WO 2016/146836

(56) Documents cités:
- WO-A1-87/07508
- WO-A1-02/094238
- WO-A1-2005/102353
- WO-A1-2011/126071
- WO-A2-2005/007082
- MARK HOLODNIY ET AL: "Influenza treatment and prophylaxis with neuraminidase inhibitors: a review", INFECTION AND DRUG RESISTANCE, 1 novembre 2013 (2013-11-01), page 187, XP055248817, DOI: 10.2147/IDR.S36601
- A. IACOANGELI ET AL: "The ionophore monensin inhibits mouse polyomavirus DNA replication and destabilizes viral early mRNAs", BIOCHIMIE, vol. 82, no. 1, 1 janvier 2000 (2000-01-01), pages 35-39, XP055274174, FR ISSN: 0300-9084, DOI: 10.1016/S0300-9084(00)00358-8
- SHOU-REN WANG ET AL: "Multiple system atrophy manifested as dizziness and imbalance: a report of two cases", EUROPEAN ARCHIVES OF OTO-RHINO-LARYNGOLOGY : OFFICIAL JOURNAL OF THE EUROPEAN FEDERATION OF OTO-RHINO-LARYNGOLOGICAL SOCIETIES (EUFOS) : AFFILIATED WITH THE GERMAN SOCIETY FOR OTO-RHINO-LARYNGOLOGY - HEAD AND NECK SURGERY, vol. 260, no. 7, 1 août 2003 (2003-08-01), pages 404-407, XP055292503, DE ISSN: 0937-4477, DOI: 10.1007/s00405-003-0595-x
- L Spahr ET AL: "Tolcapone-related fulminant hepatitis: electron microscopy shows mitochondrial alterations", Digestive diseases and sciences, vol. 45, no. 9 1 septembre 2000 (2000-09-01), pages 1881-1884, XP055292510, UNITED STATES Extrait de l'Internet: URL:http://download.springer.com/static/pd f/374/art%3A10.1023%2FA%3A1005549304404.pd f?originUrl=http://link.springer.com/artic le/10.1023/A:1005549304404&token2=exp=1470 056469~acl=/static/pdf/374/art%253A10.1023 %252FA%253A1005549304404.pdf?originUrl=htt p%3A%2F%2Flink.springer.com%2Farticle%2F10 .1023%2FA%

## Description

L'invention se rapporte à des compositions pour leur utilisation dans le traitement des infections virales liées aux virus de la grippe, chez l'homme et chez les animaux.

Les virus responsables de la grippe sont les virus influenza qu'on divise en trois types: A, B et C. A la surface du virus se trouvent deux glycoprotéines qui jouent un rôle important dans l'infection des cellules de l'organisme infecté : l'hémagglutinine (HA) et la neuraminidase (NA). Il existe différents sous-types de virus influenza A selon la nature des glycoprotéines HA et NA à leur surface: 16 types d'HA et 9 types de NA ont été identifiées chez les virus circulants dans le monde animal et notamment parmi les oiseaux migrateurs marins. On peut ainsi définir les virus influenza selon le type de glycoprotéines qu'ils possèdent à leur surface.

Chez l'homme, les virus circulants depuis plusieurs décennies sont les sous-types H1N1, H2N2 et H3N2, avec des transmissions inter-espèces occasionnelles, notamment de l'animal à l'homme, des virus aviaires H5N1, H7N7, H7N9, H5N2 et H9N2. Comme le souligne la récente émergence d'un nouveau virus grippal pandémique H1N1 d'origine porcine, aviaire et humaine (virus réassortant porcin, aviaire et humain), les virus influenza de type A représentent une menace sérieuse en santé publique. Les pandémies de grippe sont le résultat notamment de cassures antigéniques qui correspondent à l'apparition de virus dotés de nouvelles glycoprotéines de surface (HA et NA) dans la population humaine. Ces cassures permettent la transmission directe chez l'homme de virus animaux et notamment aviaires : c'est le cas des épidémies de H5N1 aviaires hautement pathogènes depuis 2003 en Asie, ou de des épidémies de grippe H7N7 aux Pays-Bas en 2003 et H7N9 dans le sud-est asiatique en 2013. Les cassures antigéniques sont le résultat de réarrangements génétiques entre des virus aviaires, porcins et humains, le porc jouant par exemple le rôle d'hôte intermédiaire. Ces réarrangements génétiques ont notamment été à l'origine de la pandémie de virus H1N1 en 2009. Par ailleurs, les épidémies de grippe saisonnières, qui sont notamment le résultat de dérive génétique (apparition de mutations dans les glycoprotéines de surface) sont une cause majeure de morbidité et de mortalité accrue, notamment dans la population humaine, surtout chez les individus très jeunes, les personnes âgées, les individus immunodéprimés et les porteurs de maladies cardio-pulmonaires.

La vaccination reste la pierre angulaire de la prévention de la grippe. Cependant, lors de l'apparition d'un nouveau virus, un délai de 6 à 9 mois est nécessaire à la mise au point et à la délivrance d'un nouveau vaccin, et l'utilisation de médicaments antiviraux doit être envisagée pour le traitement et/ou la prévention. Les antiviraux usuels sont les inhibiteurs des canaux M2 tels que l'amantadine et les inhibiteurs de la neuraminidase tels que le zanamivir et l'Oseltamivir.

L'utilisation de ces médicaments est limitée par l'apparition récurrente de résistances, notamment observées pour le virus H1N1 pandémique. De plus, il n'est pas exclu que les nouveaux virus émergents soient déjà résistants à ces molécules. Enfin, la plupart de ces molécules ne sont pas administrables par voie générale, ce qui pose un problème en cas d'infections graves.

Il apparaît donc nécessaire de mettre au point ou d'identifier de nouveaux composés antiviraux plus efficaces et possédant un large spectre d'action. Une solution pour la mise au point de nouvelles molécules thérapeutiques à large spectre est d'identifier des molécules ayant une action sur les voies et facteurs cellulaires que ciblent les virus pour mener à bien leur cycle réplicatif. Cette stratégie, présentée dans l'article de Josset et al. (Plos One, 2010), a permis l'identification d'une première série de molécules présentant une activité antivirale inattendue, telles que les molécules présentées dans la demande de brevet FR 2 953 410.

Dans le cadre de la présente invention, plusieurs molécules ont été sélectionnées et évaluées dans un test cellulaire d'infection virale ainsi que dans un modèle murin *in vivo.* Certaines molécules ont ainsi présenté un effet antiviral non seulement sur une souche influenza A de sous-type H1N1 mais également sur une souche de sous-type H3N2. Les composés sélectionnés avaient été décrits préalablement en tant que principes actifs dans le traitement de pathologies très éloignées des infections virales. De façon inattendue, il a maintenant été montré que certains de ces composés ont une activité antivirale et en particulier contre différents sous-types de virus influenza A.

### RESUME DE L'INVENTION

L'invention a pour objet des compositions pharmaceutiques ou vétérinaires pour leur utilisation dans une méthode de prévention et/ou de traitement des infections par les virus influenza, comprenant du Diltiazem et un autre agent antiviral choisi parmi l'Oseltamivir et le Zanamivir.

Ces compositions pharmaceutiques ou vétérinaires peuvent avantageusement comprendre en outre de l'Etiléfrine et/ou un agent antibactérien. Ces compositions pourront être sous la forme de produits de combinaison, pour une utilisation simultanée, séparée ou séquentielle en thérapie humaine ou vétérinaire, notamment dans une méthode de prévention et/ou de traitement des infections par les virus influenza.

L'invention a également pour objet des compositions pharmaceutiques ou vétérinaires comprenant au moins un agent antiviral choisi parmi l'Oseltamivir et le Zanamivir, en combinaison avec du Diltiazem, ou avec une combinaison de Diltiazem et d'Etiléfrine.

Selon un aspect préféré de l'invention, ces compositions pharmaceutiques ou vétérinaires sont sous une forme galénique particulière destinée à leur administration par inhalation.

### FIGURES

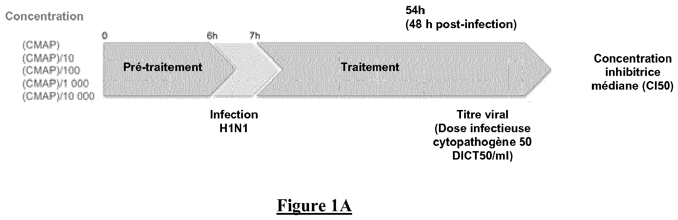

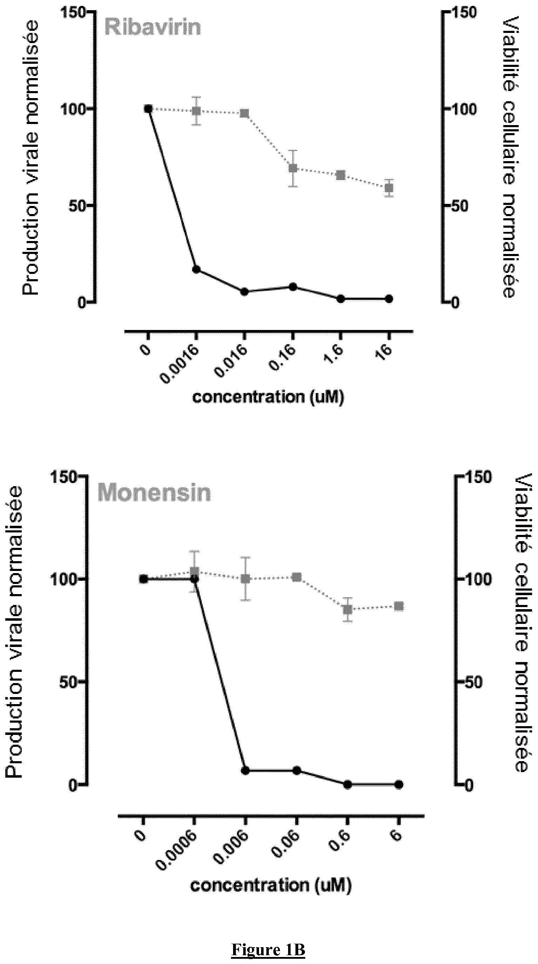

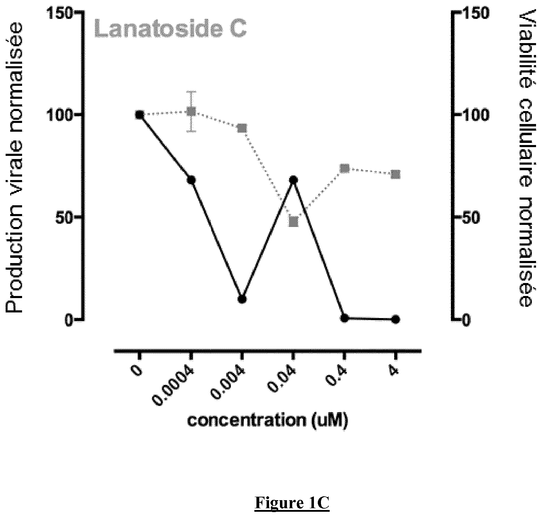

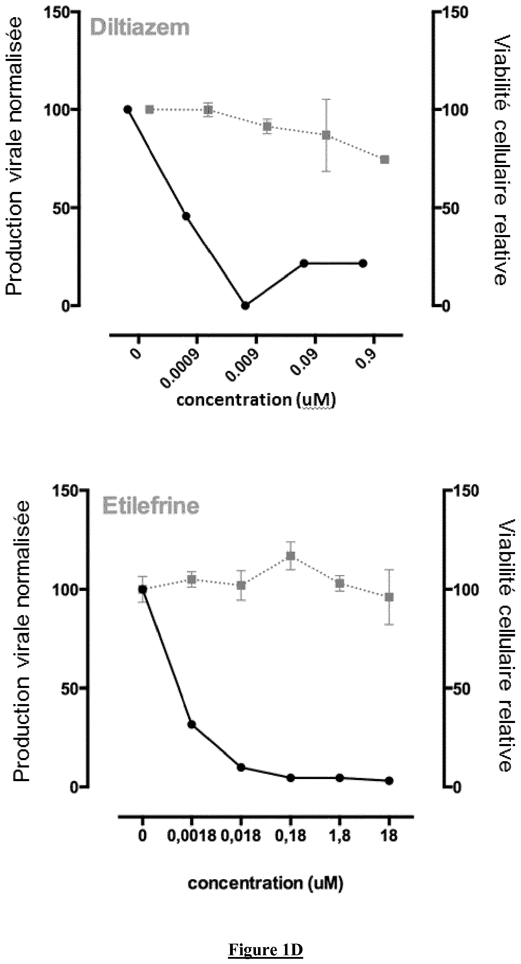

**Figure 1****.** (A) Protocole expérimental ; Evaluation *in vitro* de l'effet antiviral de composés connus pour leur activité antivirale (1B : Ribavirine, Monensin) et des composés Lanatoside C (1C) et Diltiazem et Etiléfrine (1D) sur des cellules A549 infectées par le virus H1N1 pdm09. La courbe en trait continu représente l'évolution de la production virale normalisée (pourcentage) en fonction de la concentration des composés (µM). La courbe en pointillée représente la viabilité cellulaire normalisée (pourcentage) en fonction de la concentration des composés (µM).

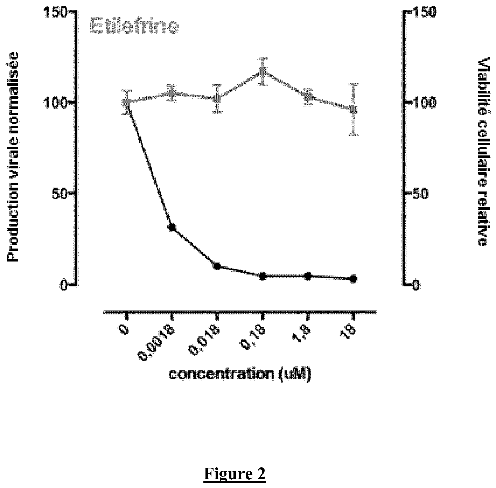

**Figure 2****.** Evaluation *in vitro* de l'effet antiviral de l'Etiléfrine sur des cellules A549 infectées par le virus H3N2. La courbe en trait continu (points ronds) représente l'évolution de la production virale normalisée (pourcentage) en fonction de la concentration d'Etilefrine (µM). La courbe en pointillée (points carrés) représente la viabilité cellulaire normalisée (pourcentage) en fonction de la concentration d'Etilefrine (µM).

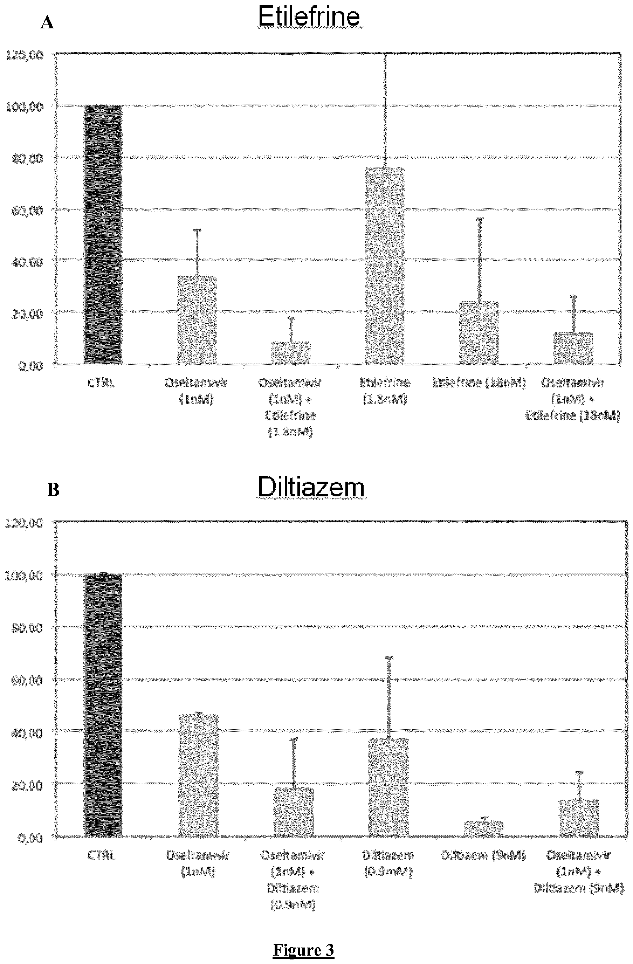

**Figure 3****.** Evaluation *in vitro* de l'effet antiviral de l'Etiléfrine (A) et du Diltiazem (B), en combinaison avec de l'Oseltamivir, sur des cellules A549 infectées par le virus H1N1 pdm09. Le graphique représente la production virale normalisée (pourcentage) pour chacune des conditions.

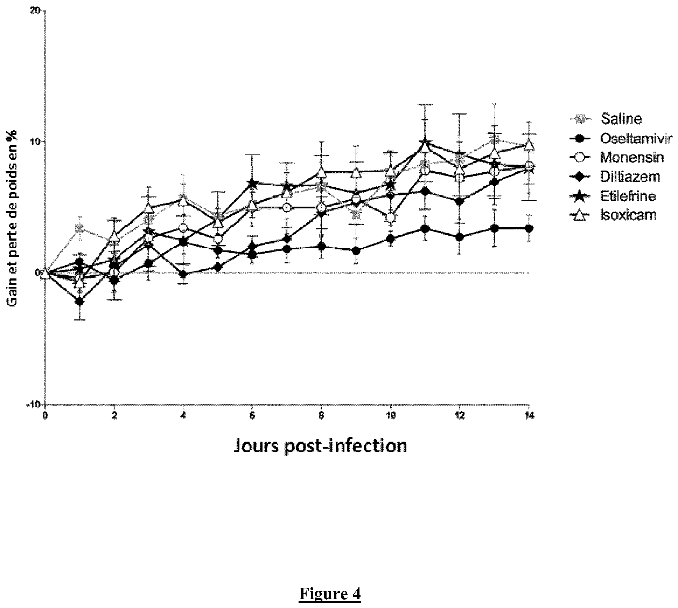

**Figure 4****.** Evaluation *in vivo* de la toxicité des molécules suivantes : Oseltamivir, Monensin, Diltiazem, Etilefrine et Isoxicam. Le témoin « Saline » indique le poids des souris traitées avec du tampon PBS. La courbe représente l'évolution du poids (Gain/perte de poids en pourcentage) des différents groupes de souris au cours du temps.

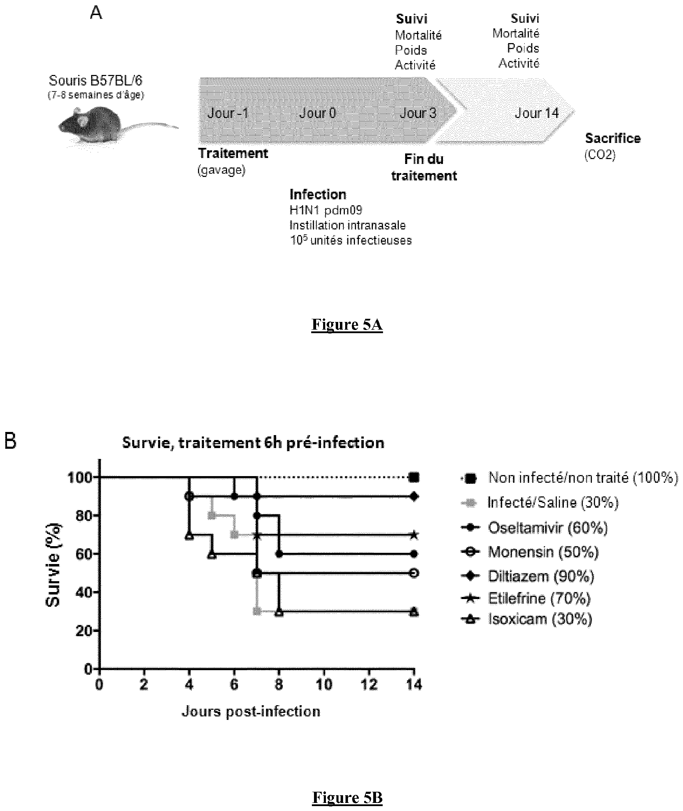

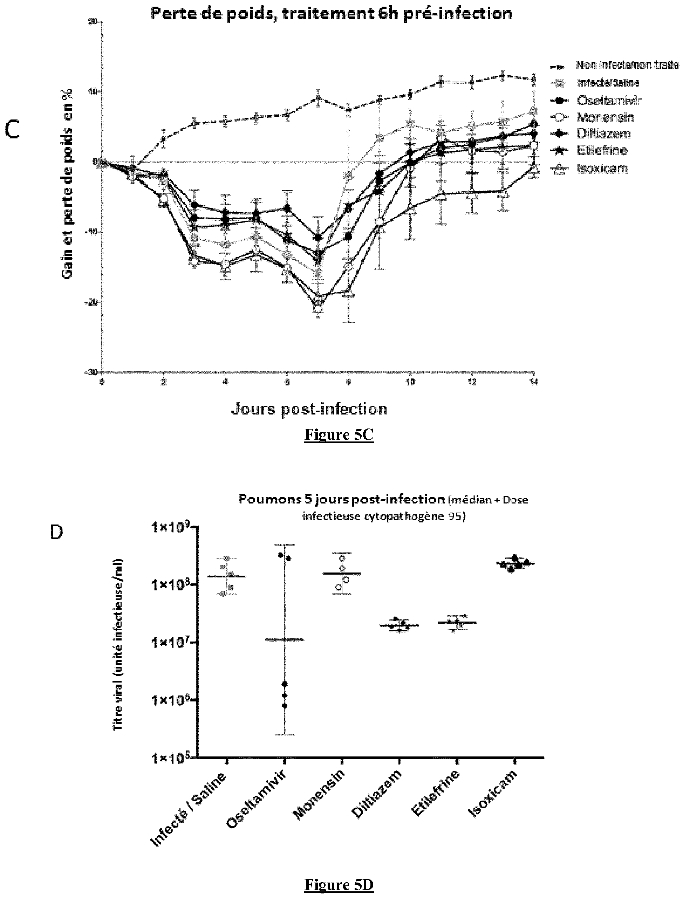

**Figure 5****.** Evaluation *in vivo* de l'effet antiviral des composés Oseltamivir, Monensin, Diltiazem, Etiléfrine et Isoxicam, administrés avant l'infection par H1N1 des souris : (A) protocole expérimental ; (B) taux de survie au cours du temps, jusqu'à 14 jours post-infection ; (C) perte de poids observée au cours du temps ; (D) Titre viral mesuré dans les poumons des souris infectées à 5 jours post-infection.

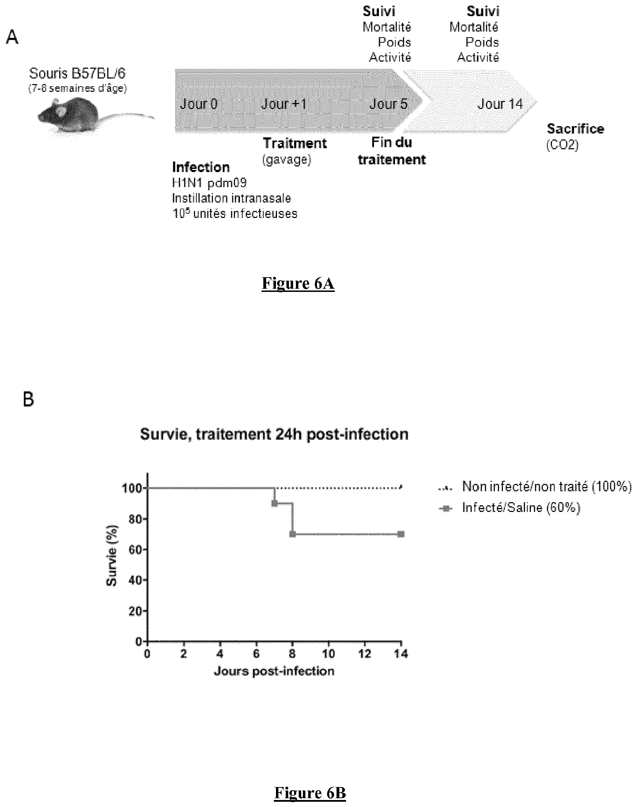

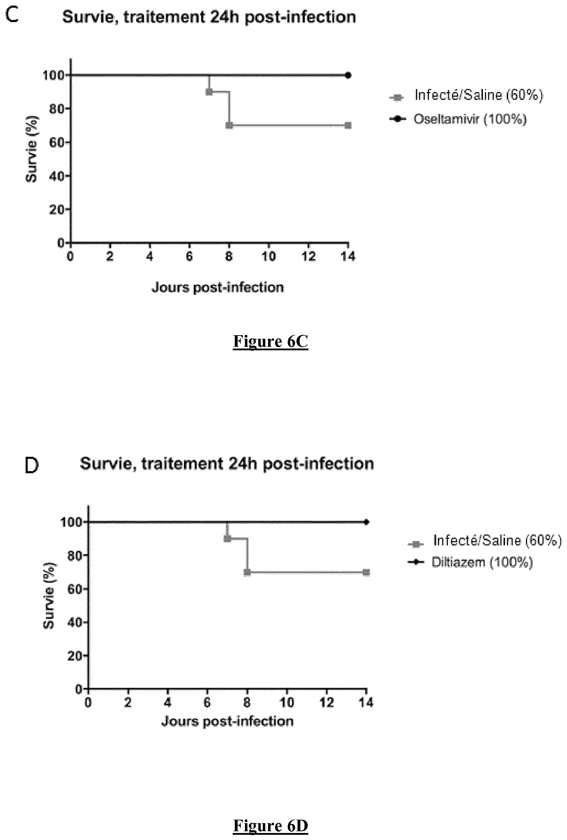

**Figure 6****.** Evaluation *in vivo* de l'effet antiviral des composés Oseltamivir et Diltiazem, administrés 24 heures après l'infection par H1N1 des souris. Protocole expérimental (A) ; Taux de survie au cours du temps, après traitement *in vivo* avec le tampon PBS seul « Saline » (B), de l'Oseltamivir (C) et du Diltiazem (D) administrés 24 h après l'infection par H1N1 des souris.

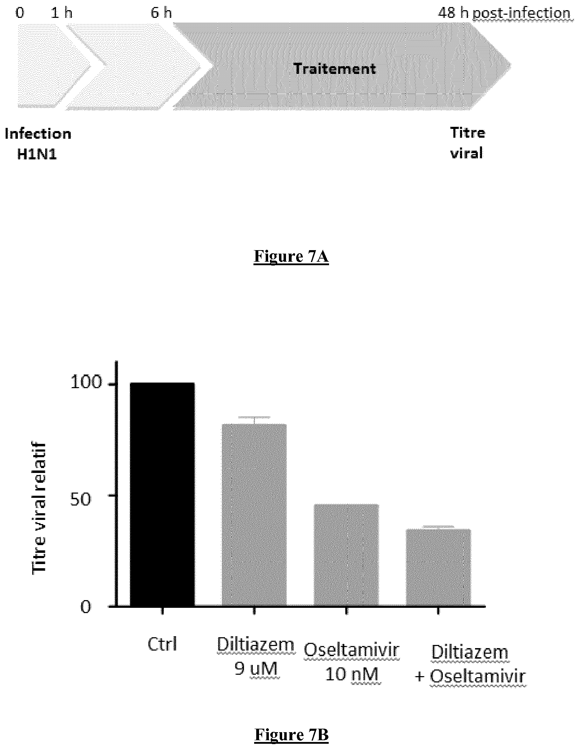

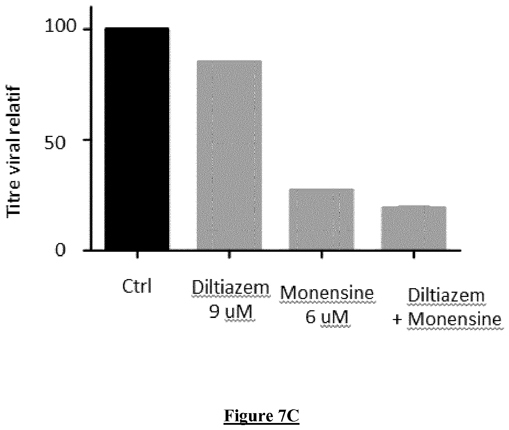

**Figure 7****.** Evaluation *in vitro* de l'effet antiviral du Diltiazem en combinaison avec d'autres agents anti-viraux, sur des cellules A549 infectées par le virus H1N1 pdm09 (A) Protocole de traitement des cellules en conditions de post-infection ; (B) Résultats obtenus pour la combinaison Diltiazem et Oseltamivir, (C) Résultats obtenus pour la combinaison Diltiazem et Monensine.

### DESCRIPTION DETAILLEE DE L'INVENTION

La présente invention a pour objet une composition pharmaceutique ou vétérinaire pour son utilisation dans dans une méthode de prévention et/ou 2. de traitement d'une infection par les virus influenza, caractérisée en ce qu'elle comprend, dans un véhicule pharmaceutique approprié, du Diltiazem et un autre agent antiviral choisi parmi l'Oseltamivir et le Zanamivir.

Ces composés sont connus pour une utilisation dans d'autres applications thérapeutiques sans rapport avec une activité antivirale contre les virus influenza chez l'homme ou chez l'animal. Il est maintenant démontré que ces composés ont de façon inattendue une activité antivirale contre différents sous-types de virus influenza de type A.

Le Diltiazem est utilisé usuellement dans la prise en charge des angors, de l'hypertension artérielle, des ischémies myocardiques, et de la tachycardie. Le Diltiazem est un puissant vasodilatateur .

Plusieurs demandes de brevet décrivent des procédés de production de Diltiazem (EP0728751, EP0561861), ainsi que des dérivés du Diltiazem (EP0450705, EP0355395).

Cette molécule, membre de la famille des benzothiazépines, présente la formule chimique développée suivante :

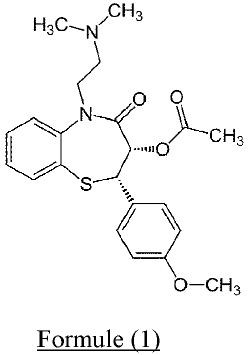

Le Diltiazem agit en freinant l'entrée du calcium transmembranaire au niveau de la fibre musculaire myocardique et de la fibre musculaire lisse des vaisseaux, et diminue ainsi la concentration intracellulaire calcique atteignant les protéines contractiles. Le Diltiazem réduit ainsi le travail cardiaque et ralentit la cadence ventriculaire.

La demande de brevet WO 87/07508 décrit l'utilisation de composés thérapeutiques inhibant les influx de calcium dans la cellule, tels que le Diltiazem, en combinaison avec d'autres agents antiviraux pour le traitement d'infections virales liées au cytomégalovirus et à l'herpès.

La demande de brevet WO 2011/126071 décrit l'utilisation de composés thérapeutiques inhibant les influx de calcium dans la cellule, tels que le Diltiazem, pour le traitement d'infections virales, notamment celles liées aux virus influenza. Plus spécifiquement, cette demande indique que le Diltiazem peut inhiber l'interaction entre un virus et les canaux calciques, et ainsi bloquer l'entrée du virus dans les cellules.

Au contraire, dans la présente invention, est mis en évidence le fait que le Diltiazem agit de manière globale sur les cellules cibles en modulant fortement l'expression des gènes de ces cellules, et en induisant ainsi un état cellulaire globalement défavorable à une infection virale. En effet, et comme présenté de manière plus détaillée dans le chapitre introductif des exemples, les molécules sélectionnées sont associées à des profils d'expression de gènes cellulaires inverses de ceux caractéristiques d'un état infectieux. De plus, WO 2011/126071 propose une concentration de Diltiazem plus de 100 fois supérieure aux concentrations proposées dans la présente demande.

Les doses de Diltiazem classiquement utilisées chez l'homme sont préférentiellement comprises entre 200 et 300 mg/jour, pour une posologie usuelle, lorsque le Diltiazem est sous forme de comprimés.

Le Diltiazem est disponible sous différentes formes galéniques, telles que de la poudre pour solution injectable (CN102657621) ou des préparations pharmaceutiques pour inhalation (WO 02/094238, US4,605,552).

Au sens de la présente invention, le terme « Diltiazem » signifie le Diltiazem sous toutes ses formes, notamment sous forme de sels, et le terme « dérivé de Diltiazem » signifie toutes les molécules dérivées de la formule (1) présentée ci-dessus, présentant la même activité biologique, notamment la même activité anti-virale, ladite activité anti-virale étant obtenue en induisant un état cellulaire globalement défavorable à une infection virale, en agissant sur l'expression d'un certain nombre de gènes des cellules cibles.

L'Etiléfrine a été décrite comme un stimulant cardiaque, et est utilisée usuellement comme agent anti-hypotensif, notamment dans la prise en charge d'hypotensions orthostatiques neurologique, cardiovasculaire, du système endocrinien ou d'origine métabolique. Son utilisation a notamment été décrite dans la demande de brevet EP0162320.

L'Etiléfrine augmente le débit cardiaque, le volume systolique, le retour veineux et élève la pression veineuse centrale et la pression artérielle, via un effet sympathomimétique direct sur les récepteurs alpha-1 et bêta adrénergiques, par son action inotrope positive. De plus, elle accroît le tonus veineux et entraîne une augmentation du volume sanguin circulant.

Il s'agit d'une amine sympathomimétique de la série 3-hydroxy-phényléthanolamine de formule chimique développée :

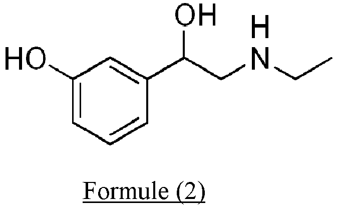

Les doses classiquement utilisées chez l'homme sont préférentiellement d'environ 30 mg/jour, généralement administrées en trois prises.

L'Etiléfrine est disponible sous différentes formes galéniques, telles que sous forme de solutions liquides injectables, solutions liquides pour administration par voie orale, et sous forme de comprimés.

Il a été également été décrit des modes d'administration par inhalation pour d'autres agonistes des récepteurs beta-adrénergiques.

Au sens de la présente invention, le terme « Etiléfrine » signifie l'Etiléfrine sous toutes ses formes, notamment sous forme de sels, et le terme « dérivés d'Etiléfrine » signifie toutes les molécules dérivées de la formule (2) présentée ci-dessus, présentant la même activité biologique, notamment la même activité antivirale, ladite activité antivirale étant obtenue en induisant un état cellulaire globalement défavorable à une infection virale, en agissant sur l'expression d'un certain nombre de gènes des cellules cibles.

Selon l'invention, les virus influenza sont des virus de type A ayant pour hôte l'homme ou l'animal. Les termes « virus de la grippe » et « virus influenza » sont utilisés indifféremment dans la demande et désigne les mêmes virus.

L'expression « infection par les virus influenza» doit se comprendre comme étant une infection générée par la présence d'au moins l'un des virus influenza connu, ce virus ayant infecté l'individu ou l'animal, ou étant susceptible d'infecter l'individu ou l'animal, à qui la composition est administrée.

Selon l'invention, le terme « véhicule pharmaceutique approprié » désigne des véhicules ou des excipients pharmaceutiquement acceptables selon l'invention, c'est à dire des véhicules ou des excipients dont l'administration à un individu ou un animal ne s'accompagne pas d'effets délétères significatifs, et qui sont bien connus de l'homme du métier.

En particulier, les compositions de la présente invention sont utilisées pour la prévention et/ou le traitement des infections par les virus influenza de type A. Avantageusement, les compositions de la présente invention ont un spectre d'action large contre les différents sous-types des virus influenza de type A.

Dans un mode de réalisation, les compositions de la présente invention sont utilisées pour la prévention et le traitement des infections par les virus de type A circulants principalement chez l'homme et les animaux.

Il existe différents sous-types de virus influenza A selon la nature des glycoprotéines HA et NA à leur surface. Selon un aspect particulier, le virus de la grippe est un virus de type A choisi parmi les sous-types H1N1, H2N2, H3N2, H5N1, H7N7, H7N9, H5N2 et H9N2.

L'invention se rapporte ainsi à la prévention et/ou au traitement des infections par les virus de la grippe chez l'homme, c'est à dire à une composition pharmaceutique pour son utilisation dans une méthode de prévention et/ou de traitement d'une infection par les virus influenza.

L'invention se rapporte aussi à la prévention et/ou au traitement des infections par les virus de la grippe chez l'animal, en particulier, chez les animaux d'élevage comme les porcs, les chevaux et les volailles. Plus particulièrement, l'invention a pour objet la prévention et/ou le traitement des infections par les virus influenza chez les volailles, et plus particulièrement chez les poules, canards, oies, dindes et dindons. L'invention se rapporte aussi à la prévention et au traitement des infections par les virus de la grippe chez d'autres animaux comme par exemple les chevaux, les chats, les chiens et les félins. Selon cet aspect, l'invention a donc trait à une composition vétérinaire pour son utilisation dans une méthode de prévention et/ou de traitement d'une infection par les virus influenza.

Les compositions selon l'invention sont en particulier destinées à une utilisation dans la prévention d'une infection par les virus influenza.

Le terme « prévention » désigne le fait d'empêcher, ou du moins de diminuer la probabilité d'apparition, d'une infection dans un organisme humain ou animal par au moins un virus influenza. Grâce à l'administration d'au moins une composition selon l'invention, les cellules humaines ou animales dudit organisme deviennent plus résistantes et sont plus à même de ne pas être infectées par ledit virus.

Les compositions selon l'invention peuvent également être destinées à une utilisation dans le traitement d'une infection par les virus influenza.

Le terme « traitement » désigne le fait de combattre l'infection par au moins un virus influenza dans un organisme humain ou animal. Grâce à l'administration d'au moins une composition selon l'invention, le taux d'infection virale dans l'organisme va peu à peu diminuer jusqu'à disparaitre complètement. Le terme «traitement» désigne aussi le fait d'atténuer les symptômes associés à l'infection virale (fièvre, fatique...).

Selon un premier aspect de l'invention, la composition pharmaceutique ou vétérinaire comprend une quantité efficace de Diltiazem et d'un autre agent antiviral choisi parmi l'Oseltamivir et le Zanamivir, pour son utilisation dans une méthode de prévention et/ou de traitement d'une infection par les virus influenza.

Selon un autre aspect de l'invention, la composition pharmaceutique ou vétérinaire comprend une combinaison de Diltiazem et d'un autre agent antiviral choisi parmi l'Oseltamivir et le Zanamivir, comprenant de plus de l'Etiléfrine, pour son utilisation dans une méthode de prévention et/ou de traitement d'une infection par un ou des virus de la grippe.

Cette combinaison comprend soit la même dose de chaque composé (composition à 50/50 en poids) soit des doses non égales de chaque composé, telles que 90 % de Diltiazem et 10 % d'Etiléfrine, 80 % de Diltiazem et 20 % d'Etiléfrine, 70 % de Diltiazem et 30 % d'Etiléfrine, 60 % de Diltiazem et 40 % d'Etiléfrine, 40 % de Diltiazem et 60 % d'Etiléfrine, 30 % de Diltiazem et 70 % d'Etiléfrine, 20 % de Diltiazem et 80 % d'Etiléfrine, ou bien encore 10 % de Diltiazem et 90 % d'Etiléfrine.

Le Diltiazem et l'Etiléfrine ou leurs mélanges dans une composition selon la revendication 1 peuvent être employés en combinaison avec au moins un autre agent actif. Il peut s'agir de composés permettant d'améliorer l'activité des composés, ou encore d'autres actifs connus pour leur emploi comme agent antiviral dans le traitement des infections par les virus influenza.

En particulier, l'agent antiviral a une activité inhibitrice directe sur les virus influenza, c'est-à-dire inhibe la réplication ou empêche la pénétration ou la propagation d'au moins un virus influenza dans un organisme infecté.

De tels agents actifs sont bien connus de l'homme du métier, disponibles dans le commerce ou encore décrits dans des ouvrages de référence comme Le Dictionnaire Vidal.

Selon un premier aspect de l'invention, la composition pharmaceutique ou vétérinaire comprend du Diltiazem et un autre agent antiviral choisi parmi l'Oseltamivir et le Zanamivir., pour son utilisation dans une méthode de prévention et/ou de traitement d'une infection par les virus influenza.

Selon un autre aspect de l'invention, la composition pharmaceutique ou vétérinaire comprend une combinaison de Diltiazem et d'un autre agent antiviral choisi parmi l'Oseltamivir et le Zanamivir, comprenant de plus de l'Etiléfrine, pour son utilisation dans une méthode de prévention et/ou de traitement d'une infection par les virus influenza.

Cet agent antiviral est choisi parmi des agents viraux ayant une action inhibitrice directe sur les virus, tels que l'Oseltamivir (aussi appelé Tamiflu) et le zanamivir. D'autres agents antiviraux connus sont le peramivir, l'amantadine, la rimantadine, la ribavirine et l'arbidol.

D'autres agents actifs sur les cellules, induisant des états cellulaires globalement défavorables à une infection virale, sont connus tels que la monensine, la midodrine, la desglymidodrine, la rilmenidine, l'harmol, les dimers d'harmol et le brinzolamide.

Un autre agent antiviral connu est la Monensine, une molécule présentant une activité de chélateur de calcium dans le milieu extracellulaire.

D'autres agents antiviraux connus sontles :
- inhibiteurs de polymérase virale (ex : T705) ;
- inhibiteurs de nucléoprotéine ;
- inhibiteurs d'hémagglutinine ;
- inhibiteurs de neuraminidase ;
- inhibiteurs de protéine NS1, M1, M2, Pb1-F2, NEP ;
Ou encore les :
- sialidases recombinantes (ex DAS181) ;
- inhibiteurs de NFKB ;
- inhibiteurs d'HSP90 ; et
- inhibiteurs de protéines kinases cellulaires Raf, Mek, Erk ou PKC.

Il est entendu que cet autre agent antiviral sera utilisé aux doses nécessaires pour présenter une action antivirale, cette dose étant désignée comme étant « efficace », ce dosage pouvant être facilement déterminé par l'homme du métier.

Selon un premier aspect préféré de l'invention, la composition pharmaceutique ou vétérinaire comprend du Diltiazem, en combinaison avec de l'Oseltamivir, pour son utilisation dans une méthode de prévention et/ou de traitement d'une infection par les virus influenza.

Selon un second aspect particulier, la composition pharmaceutique ou vétérinaire comprend du Diltiazem, en combinaison avec du Zanamivir, pour son utilisation dans une méthode de prévention et/ou de traitement d'une infection par les virus influenza.

Selon un autre aspect de l'invention, la composition pharmaceutique ou vétérinaire comprend du Diltiazem, de la Monensine et de l'Oseltamivir, pour son utilisation dans une méthode de prévention et/ou de traitement d'une infection par les virus influenza.

Selon un autre aspect de l'invention, la composition pharmaceutique ou vétérinaire comprend du Diltiazem, de la Monensine et du Zanamivir, pour son utilisation dans une méthode de prévention et/ou de traitement d'une infection par les virus influenza.

Selon un troisième aspect préféré de l'invention, la composition pharmaceutique ou vétérinaire comprend une combinaison de Diltiazem et d'Etiléfrine, en combinaison avec de l'Oseltamivir, pour son utilisation dans une méthode de prévention et/ou de traitement d'une infection par les virus influenza.

La composition pharmaceutique ou vétérinaire pour son utilisation selon l'invention peut également comprendre au moins un agent antibactérien. Un tel agent sera en particulier un antibiotique, destiné à prévenir les surinfections bactériennes classiquement observées en complications des infections par les virus influenza.

Selon un premier aspect de l'invention, la composition pharmaceutique ou vétérinaire comprend du Diltiazem et un autre agent antiviral choisi parmi l'Oseltamivir et le Zanamivir, en combinaison avec au moins un agent antibactérien, pour son utilisation dans une méthode de prévention et/ou de traitement d'une infection par les virus influenza.

Selon un autre aspect de l'invention, la composition pharmaceutique ou vétérinaire comprend une combinaison de Diltiazem et d'un autre agent antiviral choisi parmi l'Oseltamivir et le Zanamivir, et de plus de l'Etiléfrine, en combinaison avec au moins un agent antibactérien, pour son utilisation dans une méthode de prévention et/ou de traitement d'une infection par les virus influenza.

Selon un autre aspect de l'invention, la composition pharmaceutique ou vétérinaire comprend une combinaison de Diltiazem et d'Etiléfrine, en combinaison avec de l'Oseltamivir, et au moins un agent antibactérien, pour son utilisation dans une méthode de prévention et/ou de traitement d'une infection par les virus influenza.

Selon un autre aspect de l'invention, la composition pharmaceutique ou vétérinaire comprend une combinaison de Diltiazem et de Monensine, en combinaison avec un autre agent antiviral choisi parmi l'Oseltamivir et le Zanamivir, et au moins un agent antibactérien, pour son utilisation dans une méthode de prévention et/ou de traitement d'une infection par les virus influenza.

### Produit de combinaison

La présente invention concerne également un produit de combinaison comprenant du Diltiazem, et au moins un agent antiviral choisi parmi l'Oseltamivir et le Zanamivir , pour une utilisation simultanée, séparée ou séquentielle dans une méthode pour prévenir et/ou
traiter une infection par les virus influenza, chez l'homme ou chez l'animal.

Selon un mode de réalisation, ledit produit de combinaison comprend du Diltiazem et au moins de l'Oseltamivir.

Est également décrit ici un produit de combinaison comprenant de l'Etiléfrine et au moins un agent antiviral tel que de l'Oseltamivir ou de la Monensine, pour une utilisation simultanée, séparée ou séquentielle dans une méthode pour prévenir et/ou traiter une infection par
les virus influenza.

Selon un autre mode de réalisation, ledit produit de combinaison comprend une combinaison de Diltiazem et d'Etiléfrine et de l'Oseltamivir.

Selon un autre mode de réalisation, ledit produit de combinaison comprend de plus au moins un agent antibactérien.

Selon un autre mode de réalisation, ledit produit de combinaison comprend une combinaison de Diltiazem, et d'au moins un agent antiviral choisi parmi l'Oseltamivir et le Zanamivir, et d'Etiléfrine et d'au moins un agent antibactérien.

Selon un autre mode de réalisation, ledit produit de combinaison comprend du Diltiazem, au moins un agent antiviral choisi parmi l'Oseltamivir et le Zanamivir et au moins un agent antibactérien.

Les agents antiviraux, notamment ayant une action inhibitrice contre les virus influenza, sont bien connus de l'homme du métier, et sont notamment les agents suivants :
- oseltamivir, zanamivir, peramivir, amantadine, rimantadine, ribavirine et arbidol ;
- la midodrine, la desglymidodrine, la rilmenidine, l'harmol, les dimers d'harmol,le brinzolamide et la Monensine ;
- les inhibiteurs de polymérase virale (ex : T705) ;
- les inhibiteurs de nucléoprotéine ;
- les inhibiteurs d'hémagglutinine ;
- les inhibiteurs de neuraminidases ;
- les inhibiteurs de protéine NS1, M1, M2, NEP, Pb1-F2 ;.
- les sialidases recombinantes (ex DAS 181) ;
- les inhibiteurs de NFKB ;
- les inhibiteurs d'HSP90 ; et
- les inhibiteurs de protéines kinases cellulaires Raf, Mek, Erk ou PKC.

L'invention se rapporte également à une méthode thérapeutique pour la prévention et/ou le traitement d'une infection par les virus influenza (virus de la grippe) chez l'homme dans laquelle on administre à un patient une quantité de Diltiazem et d'un autre agent antiviral choisi parmi l'Oseltamivir et le Zanamivir, , optionnellement en association avec de l'Etiléfrine et/ou un composé antibactérien.

L'invention se rapporte également à une méthode thérapeutique pour la prévention et/ou le traitement d'une infection par les virus influenza chez les animaux dans laquelle on administre à un animal une quantité efficace de Diltiazem et d'un autre agent antiviral choisi parmi l'Oseltamivir et le Zanamivir, optionnellement en association avec de l'Etiléfrine et/ou un composé antibactérien. Avantageusement, l'animal est un animal d'élevage tel que par exemple, le porc, le cheval ou encore les volailles et les animaux domestiques (chiens, chats, etc.).

### Galénique des compositions pharmaceutiques ou vétérinaires

Selon un aspect préféré de l'invention, la composition pharmaceutique ou vétérinaire pour son utilisation dans une méthode de prévention et/ou de traitement d'une infection par
les virus influenza est caractérisée en ce qu'elle est sous une forme galénique destinée à une administration par inhalation.

L'inhalation désigne l'absorption par les voies respiratoires. C'est en particulier une méthode d'absorption de composés à des fins thérapeutiques, de certaines substances sous forme de gaz, de micro-gouttelettes ou de poudre en suspension.

L'administration de compositions pharmaceutiques ou vétérinaires par inhalation, c'est-à-dire par les voies nasale et/ou buccale, est bien connue de l'Homme du métier.

On distingue deux types d'administration par inhalation :
- l'administration par insufflation lorsque les compositions sont sous la forme de poudres, et
- l'administration par nébulisation lorsque les compositions sont sous la forme d'aérosols (suspensions) ou sous la forme de solutions, par exemple de solutions aqueuses, mises sous pression. L'utilisation d'un nébuliseur ou d'un pulvérisateur sera alors recommandée pour administrer la composition pharmaceutique ou vétérinaire.

La forme galénique considérée ici est donc choisie parmi : une poudre, une suspension aqueuse de gouttelettes ou une solution sous pression.

### Compositions pharmaceutiques ou vétérinaires

La présente invention concerne également une composition pharmaceutique ou vétérinaire, comprenant dans un véhicule pharmaceutique approprié, au moins un agent antiviral choisi parmi l'Oseltamivir ou le Zanamivir, en combinaison avec du Diltiazem, ou avec une combinaison de Diltiazem et d'Etiléfrine .

L'agent antiviral est choisi parmi les agents antiviraux bien connus de l'homme du métier, et classiquement utilisés pour prévenir ou traiter la grippe, notamment présentant une action inhibitrice directe contre les virus influenza, c'est-à-dire inhibant la réplication ou empêchant la pénétration ou la propagation d'au moins un virus influenza dans un organisme infecté.

L'agent antiviral est l'Oseltamivir ou le Zanamivir.
D'autres agents connus sont le Peramivir, l'Amantadine, la Rimantadine, la Ribavirine et l'Arbidol.

D'autres agents actifs sur les cellules sont connus, notamment des agents induisant des états cellulaires globalement défavorables à une infection virale, tels que la midodrine, la desglymidodrine, la rilmenidine, l'harmol, les dimers d'harmol le brinzolamide et la Monensine.

D'autres agents antiviraux connus sont les :
- inhibiteurs de polymérase virale (ex : T705) ;
- inhibiteurs de nucléoprotéine ;
- inhibiteurs d'hémagglutinine ;
- inhibiteur de la neuraminidase
- inhibiteurs de protéine NS1, M1, M2, NEP, Pb1-F2 ;
ou encore les :
• sialidases recombinantes (ex DAS181) ;
• inhibiteurs de NFKB ;
• inhibiteurs d'HSP90 ; et
• inhibiteurs de protéines kinases cellulaires Raf, Mek, Erk ou PKC.

Selon un aspect préférentiel, l'agent antiviral est l'Oseltamivir.

L'invention concerne donc spécifiquement :
- une composition pharmaceutique ou vétérinaire, comprenant dans un véhicule pharmaceutique approprié, au moins un agent antiviral choisi parmi l'Oseltamivir ou le Zanamivir, en combinaison avec du Diltiazem ; et
   ; et
- une composition pharmaceutique ou vétérinaire, comprenant dans un véhicule pharmaceutique approprié, au moins un agent antiviral choisi parmi l'Oseltamivir ou le Zanamivir, en combinaison avec de l'Etiléfrine et du Diltiazem.

Cet agent antiviral sera notamment l'Oseltamivir.

Comme indiqué précédemment, toute combinaison de Diltiazem et d'Etiléfrine comprend soit la même dose de chaque composé (composition à 50/50 en poids) soit des doses non égales de chaque composé, telles que 90 % de Diltiazem et 10 % d'Etiléfrine, 80 % de Diltiazem et 20 % d'Etiléfrine, 70 % de Diltiazem et 30 % d'Etiléfrine, 60 % de Diltiazem et 40 % d'Etiléfrine, 40 % de Diltiazem et 60 % d'Etiléfrine, 30 % de Diltiazem et 70 % d'Etiléfrine, 20 % de Diltiazem et 80 % d'Etiléfrine, ou bien encore 10 % de Diltiazem et 90 % d'Etiléfrine.

Il est entendu que, dans la présente description, tous les composés cités sont présents dans les compositions pharmaceutiques ou vétérinaires en des quantités efficaces, c'est-à-dire en des quantités permettant d'obtenir l'effet antiviral escompté. La détermination de quantités efficaces est accessible sans difficulté à l'Homme du métier.

Les compositions pharmaceutiques ou vétérinaires de la présente invention sont adaptées pour une administration orale, sublinguale, sous-cutanée, intramusculaire, intraveineuse, transdermique, oculaire, locale ou rectale, le composé actif pouvant être administré sous formes unitaires d'administration, en mélange avec des supports pharmaceutiques classiques, aux animaux ou aux êtres humains.

Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le composé actif principal avec un véhicule pharmaceutique approprié tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le composé actif avec un diluant et en versant le mélange obtenu dans des gélules molles ou dures.

Une préparation sous forme de sirop ou d'élixir peut contenir le composé actif conjointement avec un édulcorant, un antiseptique, ainsi qu'un agent donnant du goût et un colorant approprié.

Les poudres ou les granules dispersibles dans l'eau peuvent contenir le composé actif en mélange avec des agents de dispersion ou des agents mouillants, ou des agents de mise en suspension, de même qu'avec des correcteurs du goût ou des édulcorants.

Selon un aspect préféré de l'invention, la composition pharmaceutique ou vétérinaire est sous une forme galénique destinée à une administration locale, telle qu'une administration via les muqueuses des voies respiratoires, c'est-à-dire une administration par inhalation.

### EXEMPLES

### Introduction

Des études de la réponse transcriptomique cellulaire lors d'une infection par influenza ont permis de mettre en évidence les voies de signalisation sollicitées et/ou détournées lors de l'infection, *in vitro* dans des cellules en culture. Ceci a permis de caractériser des « signatures transcriptomiques » *in vitro* spécifiques de l'infection par différents virus influenza, humains et aviaires (Josset et al. PLoS One, 2010 ; Terrier et al. Virol J, 2011 ; Terrier et al. J Gen Virol, 2013).

Cette même stratégie a été appliquée à des échantillons cellulaires *in vivo,* issus des lavages nasals de 9 patients, afin de caractériser les signatures *in vivo* de l'infection par le virus H1N1 pandémique (pdm09). Ces signatures transcriptomiques ont été obtenues sur la plateforme fluidique 450 d'Affymetrix, grâce à différents outils d'analyse bio-informatique. A l'issue d'un criblage « in silico » dans des bases de données publiques telles que Connectivity Map (Broad Institute, MIT), 34 molécules ont été sélectionnées, dont entre autres l'Etiléfrine, le Diltiazem, la Monensine, le Lanatoside C, et l'Isoxicam. Ces molécules ont été évaluées pour leur activité antivirale en modèle cellulaire d'infection *in vitro,* sur la lignée de cellules A549 d'épithélium pulmonaire humain, puis *in vivo* dans un modèle murin infectieux.

**Exemple 1.** Evaluation *in vitro* de l'effet antiviral de différents composés sélectionnés sur des cellules A549 infectées par H1N1.

### Protocole détaillé de culture et d'infection :

### A. Culture des cellules humaines A549

Les cellules A549 (carcinome pulmonaire) sont maintenues en culture dans un milieu DMEM (milieu modifié Dulbecco, BioWhittaker) complémenté avec 10 % (v/v) de sérum de veau foetal, 2 mM de L- glutamine, 100 U/mL de pénicilline et 100 µg/mL de streptomycine, dans un incubateur à 37 °C, à une atmosphère saturée en humidité contenant 5 % de CO2. A confluence, les cellules sont détachées du support par traitement à la trypsine-EDTA et réensemencées dans une fiole de culture contenant 15 ml de milieu frais. Trois jours avant l'étape d'infection, 0,75 10⁶ cellules sont réparties par flasques 25 cm² (T25), en milieu à 10 % de sérum de veau foetal de façon à obtenir 70-80 % de confluence lors de l'infection.

### B. Infection des cellules par des virus influenza représentatifs

Les cellules sont infectées par les différents virus influenza à une multiplicité d'infection (moi) de 0,1 dans du DMEM, 2 mM de L- glutamine, 100 U/mL de pénicilline et 100 µg/mL de streptomycine, et 0,5 µg/mL de trypsine.

Les virus utilisés sont : les souches de virus influenza A humains (H1Nl A/Pdm/09) et H3N2 (H3N2 A/Moscow/10/99)

### C. Pré-traitement et traitement

Les molécules ont été testées sur les cellules A549 à 70-80 % de confluence. Les cellules sont incubées pendant 6 h avec différentes concentrations de molécules, puis ont été infectées par les différents virus pendant 1h, et ont été remises en présences des molécules, aux mêmes concentrations.

### D. Mesures

Un test de cytotoxicité et un test de quantification du virus sont réalisés après 48 h d'incubation à 37 °C sous 5 % de CO2. La cytotoxicité des différentes molécules est déterminée à chaque essai dans une plaque de cellules non infectées par un test de viabilité (Test MTS, Promega). Ce test est basé sur la mesure de l'activité métabolique des cellules, qui transforme un substrat (MTS tetrazolium) en un produit (Formazan), soluble dans le milieu et dont l'absorbance mesurée à 490 nm reflète proportionnellement le nombre de cellules vivantes. Le rapport de l'absorbance dans chaque puits sur l'absorbance moyenne des puits des cellules témoins (non traitées par les molécules) est calculé et indiqué sur les schémas comme un indice de viabilité cellulaire (viabilité cellulaire relative).

L'effet sur la production virale est estimé par détermination des titres infectieux (DITC50/mL, dose infectieuse 50%) réalisée en cellules MDCK, Les titres étant calculés selon la technique de Reed et Muench. Le rapport du titre infectieux dans chaque condition a été exprimé en fonction du titre infectieux mesuré dans la condition témoin

### E. Résultats

Le protocole de pré-traitement et traitement des cellules est schématisé en figure 1A. Plusieurs concentrations ont été testées. Les cellules sont pré-traitées pendant 6 heures avec les différentes molécules testées, puis sont soumises à une infection par le virus H1N1 pdm09, pendant une heure. Le traitement avec les différentes molécules testées est alors repris pendant 48 heures. Les mesures effectuées à la fin du traitement telles que décrites précédemment permettent de déterminer la concentration inhibitrice médiane (CI50), dépendante de la production virale en fonction des différentes concentrations testées.

La figure 1B montre les résultats obtenus pour les molécules suivantes : Ribavirine, Monensine, Lanatoside C, Diltiazem et Etiléfrine. Comme attendu, les composés connus pour leur activité antivirale : la Ribavirine et la Monensine, induisent une baisse significative de la production virale normalisée. De manière plus surprenante, le Diltiazem et l'Etiléfrine présentent également une baisse significative de la production virale normalisée. Parmi les composés sélectionés comme décrit en introduction, le Lanatoside C a été testé pour évaluer son activité antivirale - les résultats obtenus n'ont pas permis de confirmer l'activité antivirale de ce composé.

**Exemple 2.** Evaluation *in vitro* de l'effet antiviral de l'Etilefrine sur des cellules A549 infectées par le sous-type viral H3N2.

Le protocole de pré-traitement et traitement est schématisé en figure 1A, et les conditions expérimentales sont les mêmes que celles décrites à l'exemple 1. Le virus H3N2 utilisé pour l'infection est : la souche A/Moscow/10/99 (H3N2).

La figure 2 montre que les effets antiviraux de l'Etiléfrine sont significatifs dès la plus faible concentration testée et permettent d'inhiber totalement la production virale à 0,018 µM d'Etiléfrine.

**Exemple 3.** Evaluation *in vitro* de l'effet antiviral de (A) l'Etilefrine et (B) du Diltiazem, en combinaison avec de l'Oseltamivir, sur des cellules A549 infectées par le virus H1N1 pdm09.

Le protocole de pré-traitement et traitement est schématisé en figure 1A, et les conditions expérimentales sont les mêmes que celles décrites à l'exemple 1.

Comme attendu, l'Oseltamivir permet de diminuer significativement la production virale aux alentours de 40 % (figures 3A et 3B). Les combinaisons Etiléfrine et Oseltamivir sont nettement plus performantes que les composés seuls, et ce aux deux concentrations d'Etiléfrine testées.

La combinaison d'Oseltamivir et Diltiazem 0,9 nM est plus efficace que les composés seuls. Les meilleurs résultats sont obtenus avec le Diltiazem seul à la concentration de 9 nM.

**Exemple 4.** Evaluation *in vivo* sur des souris de la toxicité des molécules suivantes : Oseltamivir, Monensine, Diltiazem, Etiléfrine et Isoxicam.

Le témoin « Saline » indique les souris non-traitées, ayant été traitées avec du tampon PBS.

Les quantités journalières suivantes ont été administrées par gavage aux souris :
- Oseltamivir : 10 mg/kg/jour
- Etiléfrine : 3 mg/kg/jour
- Diltiazem : 90 mg/kg/j our

Le composé ayant la plus forte toxicité cellulaire est l'Oseltamivir, cette toxicité étant néanmoins acceptable, les souris maintenant un gain de poids positif au cours du traitement. Le Diltiazem (losange) et l'Etiléfrine (étoile) sont clairement non toxiques aux doses administrées.

**Exemple 5.** Evaluation *in vivo* de l'effet antiviral des composés Oseltamivir, Monensine, Diltiazem, Etiléfrine et Isoxicam, sur des souris B57BL/6 infectées : (A) Protocole d'administration ; (B) taux de survie au cours du temps, jusqu'à 14 jours post-infection ; (C) perte de poids observée au cours du temps ; (D) Titre viral dans les poumons.

Les souris B57BL/6 à 7-8 semaines d'âge sont traitées par gavage avant (jour -1), le jour même et les trois jours suivant l'infection par le virus H1N1 pdm09, selon le protocole schématisé en figure 5A.

Les paramètres suivants sont évalués au cours du temps :
Fig 5B : taux de survie au cours du temps, jusqu'à 14 jours post-infection ;
Fig 5C : perte et gain de poids observés au cours du temps ;
Fig 5D : Titre viral dans les poumons des souris infectées et traitées.

L'Etiléfrine (représentée par des étoiles) et le Diltiazem (représenté par des losanges) permettent une survie maximale des souris infectées, soit respectivement 70 % et 90 % de survie à 14 jours, ce qui est un meilleur résultat que celui obtenu pour l'Oseltamivir (représenté par les ronds, 60 % de survie).

**Exemple 6.** Evaluation *in vivo* de l'effet antiviral des composés Oseltamivir et Diltiazem administrés 24 h après l'infection des souris par le virus H1N1 pdm09 : taux de survie au cours du temps, jusqu'à 14 jours post-infection.

Les souris B57BL/6 à 7-8 semaines d'âge sont traitées par gavage après l'infection par le virus H1N1 pdm09, selon le protocole schématisé en figure 6A. Le taux de survie au cours du temps est mesuré pour les souris infectées, non traitées (Fig. 6B) ; pour les souris traitées à l'Oseltamivir (Fig. 6C) ; et pour les souris traitées au Diltiazem (Fig. 6D). Le taux de survie des souris traitées à l'Oseltamivir, et de celles traitées au Diltiazem, est de 100 %.

**Exemple 7.** Evaluation *in vitro* de l'effet antiviral du Diltiazem en combinaison avec de l'Oseltamivir, sur des cellules A549 infectées par le virus H1N1 pdm09 (figure 7).

Le protocole de traitement des cellules en conditions de post-infection est schématisé en figure 7A. Les cellules sont soumises à une infection par le virus H1N1 pdm09, pendant une heure, puis traitées à partir de 6 heures post-infection pendant 48 heures, ou non traitées (mesure contrôle : Ctrl). Les mesures effectuées à la fin du traitement, telles que décrites précédemment, permettent de déterminer l'effet des traitements sur la production virale.

Les figures 7B et 7C montrent les résultats obtenus pour les combinaisons Diltiazem et Oseltamivir, et Diltiazem et Monensine, respectivement.

En condition de traitement en post-infection, l'Oseltamivir (10 nM) permet de diminuer significativement la production virale à une valeur diminuée d'environ 55 % par rapport à la valeur contrôle, et le Diltiazem (9 µM) permet de diminuer significativement le titre viral relatif d'environ 20 % par rapport à la valeur contrôle.

La combinaison Diltiazem et Oseltamivir est nettement plus performante que les composés seuls, car elle permet de diminuer significativement la production virale d'environ 70 % par rapport à la valeur contrôle. Ainsi, les effets de l'Oseltamivir sont potentialisés par la présence du Diltiazem.

En condition de traitement en post-infection, la Monensine (6 µM) est extrêmement efficace puisqu'elle permet de diminuer significativement la production virale d'environ 72 % par rapport à la valeur contrôle. Comme déjà indiqué, le Diltiazem (9 µM) permet de diminuer la production virale d'environ 20 %.

La combinaison de Diltiazem et de la Monensine est plus efficace que les composés seuls, car elle permet de diminuer significativement la production virale d'environ 82 % par rapport à la valeur contrôle. Ainsi, les effets de la Monensine sont potentialisés par la présence du Diltiazem.

### REFERENCES

### Brevets

- FR 2 953 410
- EP0162320
- EP0728751
- EP0561861
- EP0450705
- EP0355395
- WO 87/07508
- WO 2011/126071
- CN102657621
- WO 02/094238
- US4,605,552

### Littérature scientifique

- Josset L, Textoris J, Loriod B, Ferraris O, Moules V, Lina B, N'guyen C, Diaz JJ, Rosa-Calatrava M. « Gene expression signature-based screening identifies new broadly effective influenza a antivirals. » PLoS One. 2010 Oct 4;5(10).
- Terrier O, Josset L, Textoris J, Marcel V, Cartet G, Ferraris O, N'guyen C, Lina B, Diaz JJ, Bourdon JC, Rosa-Calatrava M. « Cellular transcriptional profiling in human lung épithélial cells infected by different subtypes of influenza A viruses reveals an overall down-regulation of the hostp53 pathway. » Virol J. 2011 Jun 8;8:285.
- Terrier O, Textoris J, Carron C, Marcel V, Bourdon JC, Rosa-Calatrava M. « Host microRNA molecular signatures associated with human H1N1 and H3N2 influenza A viruses reveal an unanticipated antiviral activity for miR-146a. » J Gen Virol. 2013 May;94(Pt 5):985-95.

## Revendications

1. Composition pharmaceutique ou vétérinaire pour son utilisation dans une méthode de prévention et/ou de traitement d'une infection par les virus influenza, **caractérisée en ce qu'**elle comprend, dans un véhicule pharmaceutique approprié, du Diltiazem et un autre agent antiviral choisi parmi l'Oseltamivir et le Zanamivir.

2. Composition pharmaceutique ou vétérinaire pour son utilisation selon la revendication 1, **caractérisée en ce que** les virus influenza sont choisis parmi les virus de type A, tels que les sous-types H1N1, H2N2, H3N2, H5N1, H7N7 et H7N9.

3. Composition pharmaceutique ou vétérinaire pour son utilisation selon l'une des revendications 1 à 2, **caractérisée en ce qu'**elle est sous une forme galénique destinée à une administration par inhalation.

4. Composition pharmaceutique ou vétérinaire pour son utilisation selon l'une des revendications 1 à 3, **caractérisée en ce qu'**elle comprend de plus de l'Etiléfrine.

5. Composition pharmaceutique ou vétérinaire pour son utilisation selon l'une des revendications 1 à 4, **caractérisée en ce que** l'agent antiviral est l'Oseltamivir.

6. Composition pharmaceutique ou vétérinaire pour son utilisation selon l'une des revendications 1 à 4, **caractérisée en ce que** l'agent antiviral est le Zanamivir.

7. Composition pharmaceutique ou vétérinaire pour son utilisation selon l'une des revendications 1 à 6, **caractérisée en ce qu'**elle comprend en outre au moins un agent antibactérien.

8. Produit de combinaison comprenant du Diltiazem, et au moins un agent antiviral choisi parmi l'Oseltamivir et le Zanamivir, pour utilisation simultanée, séparée ou séquentielle dans une méthode pour prévenir et/ou traiter une infection par les virus influenza.

9. Produit de combinaison pour utilisation selon la revendication 8 **caractérisé en ce que** l'agent antiviral est l'Oseltamivir.

10. Produit de combinaison pour utilisation selon la revendication 8 **caractérisé en ce que** l'agent antiviral est le Zanamivir.

11. Produit de combinaison pour utilisation selon la revendication 8, comprenant du Diltiazem, de l'Oseltamivir et au moins un agent antibactérien.

12. Produit de combinaison pour utilisation selon la revendication 8, comprenant du Diltiazem, du Zanamivir et au moins un agent antibactérien.

13. Composition pharmaceutique ou vétérinaire, comprenant dans un véhicule pharmaceutique approprié, au moins un agent antiviral choisi parmi l'Oseltamivir ou le Zanamivir,
en combinaison avec du Diltiazem, ou avec une combinaison de Diltiazem et d'Etiléfrine.

14. Composition pharmaceutique ou vétérinaire selon la revendication 13, dans laquelle l'agent antiviral est l'Oseltamivir.

15. Composition pharmaceutique ou vétérinaire selon la revendication 13, dans laquelle l'agent antiviral est le Zanamivir.

16. Composition pharmaceutique ou vétérinaire selon l'une des revendications 13 à 15, **caractérisée en ce qu'**elle est sous une forme galénique destinée à une administration par inhalation.

## Patentansprüche

1. Pharmazeutische oder veterinärmedizinische Zusammensetzung für ihre Verwendung bei einer Methode zur Vorbeugung und/oder Behandlung einer Infektion durch die Influenzaviren,
**dadurch gekennzeichnet, dass** sie in einem pharmazeutisch geeigneten Träger, Diltiazem und ein anderes antivirales Mittel umfasst, das aus Oseltamivir und Zanamivir ausgewählt ist.

2. Pharmazeutische oder veterinärmedizinische Zusammensetzung für ihre Verwendung nach Anspruch 1, **dadurch gekennzeichnet, dass** die Influenzaviren aus den Viren von Typ A wie den Untertypen H1N1, H2N2, H3N2, H5N1, H7N7 und H7N9 ausgewählt sind.

3. Pharmazeutische oder veterinärmedizinische Zusammensetzung für ihre Verwendung nach einem der Ansprüche 1 bis 2, **dadurch gekennzeichnet, dass** sie in einer galenischen Form vorliegt, die für eine Verabreichung durch Inhalation bestimmt ist.

4. Pharmazeutische oder veterinärmedizinische Zusammensetzung für ihre Verwendung nach einem der Ansprüche 1 bis 3, **dadurch gekennzeichnet, dass** sie zusätzlich Etilefrin umfasst.

5. Pharmazeutische oder veterinärmedizinische Zusammensetzung für ihre Verwendung nach einem der Ansprüche 1 bis 4, **dadurch gekennzeichnet, dass** das antivirale Mittel Oseltamivir ist.

6. Pharmazeutische oder veterinärmedizinische Zusammensetzung für ihre Verwendung nach einem der Ansprüche 1 bis 4, **dadurch gekennzeichnet, dass** das antivirale Mittel Zanamivir ist.

7. Pharmazeutische oder veterinärmedizinische Zusammensetzung für ihre Verwendung nach einem der Ansprüche 1 bis 6, **dadurch gekennzeichnet, dass** sie ferner mindestens ein antibakterielles Mittel umfasst.

8. Kombinationsprodukt, umfassend Diltiazem und mindestens ein antivirales Mittel, das aus Oseltamivir und Zanamivir ausgewählt ist,
für die gleichzeitige, getrennte oder sequentielle Verwendung bei einer Methode zur Vorbeugung und/oder Behandlung einer Infektion durch die Influenzaviren.

9. Kombinationsprodukt zur Verwendung nach Anspruch 8, **dadurch gekennzeichnet, dass** das antivirale Mittel Oseltamivir ist.

10. Kombinationsprodukt zur Verwendung nach Anspruch 8, **dadurch gekennzeichnet, dass** das antivirale Mittel Zanamivir ist.

11. Kombinationsprodukt zur Verwendung nach Anspruch 8, umfassend Diltiazem, Oseltamivir und mindestens ein antibakterielles Mittel.

12. Kombinationsprodukt zur Verwendung nach Anspruch 8, umfassend Diltiazem, Zanamivir und mindestens ein antibakterielles Mittel.

13. Pharmazeutische oder veterinärmedizinische Zusammensetzung, umfassend in einem geeigneten pharmazeutischen Träger mindestens ein antivirales Mittel, das aus Oseltamivir oder Zanamivir ausgewählt ist,
in Kombination mit Diltiazem oder mit einer Kombination aus Diltiazem und Etilefrin.

14. Pharmazeutische oder veterinärmedizinische Zusammensetzung nach Anspruch 13, wobei das antivirale Mittel Oseltamivir ist.

15. Pharmazeutische oder veterinärmedizinische Zusammensetzung nach Anspruch 13, wobei das antivirale Mittel Zanamivir ist.

16. Pharmazeutische oder veterinärmedizinische Zusammensetzung nach einem der Ansprüche 13 bis 15, **dadurch gekennzeichnet, dass** sie in einer galenischen Form vorliegt, die für eine Verabreichung durch Inhalation bestimmt ist.

## Claims

1. A pharmaceutical or veterinary composition for the use thereof in a method for preventing and/or treating an infection by influenza viruses, **characterized in that** it comprises, in a suitable pharmaceutical vehicle, Diltiazem and another antiviral agent selected from Oseltamivir and Zanamivir.

2. The pharmaceutical or veterinary composition for the use thereof according to claim 1, **characterized in that** the influenza viruses are selected from type A viruses, such as the H1N1, H2N2, H3N2, H5N1, H7N7 and H7N9 subtypes.

3. The pharmaceutical or veterinary composition for the use thereof according to one of claims 1 to 2, **characterized in that** it is in a dosage form intended for administration by inhalation.

4. The pharmaceutical or veterinary composition for the use thereof according to one of claims 1 to 3, **characterized in that** it also comprises Etilefrine.

5. The pharmaceutical or veterinary composition for the use thereof according to one of claims 1 to 4, **characterized in that** the antiviral agent is Oseltamivir.

6. The pharmaceutical or veterinary composition for the use thereof according to one of claims 1 to 4, **characterized in that** the antiviral agent is Zanamivir.

7. The pharmaceutical or veterinary composition for the use thereof according to one of claims 1 to 6, **characterized in that** it further comprises at least one antibacterial agent.

8. A combination product comprising Diltiazem, and at least one antiviral agent selected from Oseltamivir and Zanamivir, for simultaneous, separate or sequential use in a method for preventing and/or treating infection by influenza viruses.

9. The combination product for use according to claim 8 **characterized in that** the antiviral agent is Oseltamivir.

10. The combination product for use according to claim 8 **characterized in that** the antiviral agent is Zanamivir.

11. The combination product for use according to claim 8, comprising Diltiazem, Oseltamivir and at least one antibacterial agent.

12. The combination product for use according to claim 8, comprising Diltiazem, Zanamivir and at least one antibacterial agent.

13. The pharmaceutical or veterinary composition, comprising in a suitable pharmaceutical vehicle, at least one antiviral agent selected from Oseltamivir or Zanamivir, in combination with Diltiazem, or with a combination of Diltiazem and Etilefrine.

14. The pharmaceutical or veterinary composition according to claim 13, wherein the antiviral agent is Oseltamivir.

15. The pharmaceutical or veterinary composition according to claim 13, wherein the antiviral agent is Zanamivir.

16. The pharmaceutical or veterinary composition according to one of claims 13 to 15, **characterized in that** it is in a dosage form intended for administration by inhalation.
